# FASCICULE DE BREVET EUROPEEN

(11) **EP 1 214 452 B1**
(45) Date de publication et mention de la délivrance du brevet: **28.06.2006**
(21) Numéro de dépôt: 00964330.5
(22) Date de dépôt: 20.09.2000
(51) Int. Cl.: C12Q 1/68

(54) **PROCEDE D'IDENTIFICATION DE NOUVEAUX GENES IMPLIQUES DANS LA REGULATION DE L'ANGIOGENESE, ETUDE DE CES GENES ET LEUR UTILISATION A DES FINS THERAPEUTIQUES**
VERFAHREN ZUR IDENTIFIZIERUNG NEUARTIGER GENE, DIE BEI DER REGULATION DER ANGIOGENESE EINER ROLLE SPIELEN, ANALYSE DIESER GENE SOWIE IHRE VERWENDUNG ZU THERAPEUTISCHEN ZWECKEN
METHOD FOR IDENTIFYING NOVEL GENES INVOLVED IN THE REGULATION OF ANGIOGENESIS, STUDY OF SAID GENES AND USE THEREOF FOR THERAPEUTIC PURPOSES

(30) Priorité: 21.09.1999 FR 9911790
(43) Date de publication de la demande: 19.06.2002
(73) Titulaire: GENE SIGNAL INTERNATIONAL SA, Epalinges, VD (CH)
(72) Inventeur: AL-MAHMOOD, Salman, Paris 75014 (FR)
(74) Mandataire: Nargolwalla, Cyra
(86) Numéro de dépôt international: PCT/FR2000/002607
(87) Numéro de publication internationale: WO 2001/021831

(56) Documents cités:
- WO-A-95/23968
- PRÖLS F ET AL.: "Differential expression of osteopontin, PC4, and CEC5, a novel mRNA species, during in vitro angiogenesis" EXPERIMENTAL CELL RESEARCH, vol. 239, 1998, pages 1-10, XP000949306
- GHO Y S ET AL.: "Development of antiangiogenin peptide using a phage-displayed peptide library" CANCER RESEARCH, vol. 57, 1997, pages 3733-3740, XP002148992 cité dans la demande
- ZIMRIN A B ET AL: "AN ANTISENSE OLIGONUCLEOTIDE TO THE NOTCH LIGAND JAGGED ENHANCES FIBROLAST GROWTH FACTOR-INDUCED ANGIOGENESIS IN VITRO*" JOURNAL OF BIOLOGICAL CHEMISTRY,US,AMERICAN SOCIETY OF BIOLOGICAL CHEMISTS, BALTIMORE, MD, vol. 271, no. 51, 20 décembre 1996 (1996-12-20), pages 32499-32502, XP002913174 ISSN: 0021-9258
- MONTESANO R ET AL.: "Basic fibroblast growth factor induces angiogenesis in vitro" PROCEEDINGS OF THE NATIONAL ACADEMY OF SCIENCES USA, vol. 83, 1986, pages 7297-7301, XP002148993 cité dans la demande
- SHIMA D T ET AL.: "Alterations in gene expression associated with changes in the state of endothelial differentiation" DIFFERENTIATION, vol. 58, 1995, pages 217-226, XP000949305
- CHOUDHURI R ET AL: "An angiogenic role for the neurokines midkine and pleiotrophin in tumorigenesis" CANCER RESEARCH,US,AMERICAN ASSOCIATION FOR CANCER RESEARCH, BALTIMORE, MD, vol. 57, 1 mai 1997 (1997-05-01), pages 1814-1819, XP002122390 ISSN: 0008-5472 cité dans la demande
- TANAKA T ET AL: "VIRAL VECTOR-TARGETED ANTIANGIOGENIC GENE THERAPY UTILIZING AN ANGIOSTATIN COMPLEMENTARY DNA" CANCER RESEARCH,US,AMERICAN ASSOCIATION FOR CANCER RESEARCH, BALTIMORE, MD, vol. 58, no. 15, 1 août 1998 (1998-08-01), pages 3362-3369, XP000857409 ISSN: 0008-5472 cité dans la demande
- MAJEWSKI S ET AL.: "Interleukin-12 inhibits angiogenesis induced by tumor cell lines in vivo" JOURNAL OF INVESTIGATIVE DERMATOLOGY, vol. 106, 1996, pages 1114-1118, XP000949299 cité dans la demande

## Description

La présente invention a pour objet l'identification de nouveaux gènes codant pour des constituants cellulaires impliqués dans la régulation de l'angiogenèse. Elle concerne plus particulièrement un procédé permettant l'identification de ces gènes. Les facteurs codés par les gènes identifiés pour l'étude clinique du processus de l'angiogenèse, pour le diagnostic et pour le traitement de pathologies liées à ce processus ainsi que pour des essais pharmacologiques, pharmacogénomiques, ou pharmacosignalitiques.

L'angiogenèse est un processus fondamental par lequel de nouveaux vaisseaux sanguins se forment. Ce processus est essentiel dans plusieurs phénomènes physiologiques normaux tels que la reproduction, le développement ou encore la cicatrisation. Dans ces phénomènes biologiques normaux, l'angiogenèse est sous contrôle strict, c'est-à-dire qu'elle est déclenchée pendant une période courte, quelques jours, puis complètement inhibée. Cependant, plusieurs pathologies sont liées à une angiogenèse invasive et incontrôlée. L'arthrite, par exemple, est une pathologie due à l'endommagement des cartilages par les néovaisseaux invasifs. Dans la rétinopathie diabétique, l'invasion de la rétine par les néovaisseaux conduit à l'aveuglement des malades; la néovascularisation de l'appareil oculaire présente la cause majeure de l'aveuglement et cette néovascularisation domine une vingtaine de maladies de l'oeil. Enfin, la croissance et la métastase des tumeurs sont directement liées à la néovascularisation et sont dépendantes de l'angiogenèse. La tumeur stimule la croissance des néovaisseaux pour sa croissance elle-même. De plus, ces néovaisseaux présentent les voies d'échappement des tumeurs pour joindre la circulation sanguine et provoquer les métastases dans des sites à distance comme le foie, le poumon ou l'os.

Dans d'autres pathologies telles que les maladies cardio-vasculaires, les maladies d'artères périphériques, les lésions vasculaires ou cérébrales, l'angiogenèse peut présenter une base thérapeutique importante. En effet, la promotion de l'angiogenèse dans les endroits endommagés peut conduire à la formation de néovaisseaux sanguins latéraux et alternatifs aux vaisseaux endommagés fournissant ainsi le sang et par conséquence l'oxygène et d'autres facteurs nutritifs et biologiques nécessaires aux survies des tissus concernés.

La formation de néovaisseaux par les cellules endothéliales implique la migration, la croissance, et la différentiation des cellules endothéliales. La régulation de ces phénomènes biologiques est directement liée à l'expression génétique. En matière d'angiogenèse, un nombre sans cesse grandissant d'études montre que la régulation de l'angiogenèse se fait à travers un équilibre entre des facteurs agissant directement sur la cellule endothéliale. Ces facteurs peuvent être stimulants, d'une part, tels que le VEGF, le FGFs, l'IL-8, le HGF/SF, le PDGF, etc... Ils peuvent aussi être inhibants, comme l'IL-10, l'IL-12, le gro-α et β, le facteur plaquettaire 4, l'angiostatine, l'inhibiteur dérivé de chondrocyte humaine, la thrombospondine, le facteur inhibiteur de la leucémie, etc....(Jensen, Surg. Neural., 1998, 49, 189-195 ; Tamatani et al., Carcinogenesis, 1999, 20, 957-962 ; Tanaka et al., Cancer Res., 1998, 58, 3362-3369 ; Ghe et al., Cancer Res., 1997, 57, 3733-3740 ; Kawahara et al., Hepatology, 1998, 28, 1512-1517 ; Chandhuni et al., Cancer Res., 1997, 57, 1814-1819 ; Jendraschak et Sage, Semin. Cancer Biol., 1996, 7, 139-146 ; Majewski et al., J. Invest. Dermatol., 1996, 106, 1114-1119).

Le contrôle de l'angiogénèse représente donc un axe stratégique, à la fois de recherche fondamentale, afin d'améliorer notre compréhension des nombreux phénomènes pathologiques liés à l'angiogénèse, mais aussi une base pour l'élaboration des nouvelles thérapies destinées à traiter les pathologies liées à l'angiogénèse.

Afin de contrôler l'angiogenèse, plusieurs groupes pharmaceutiques ont développé des stratégies thérapeutiques basées directement sur l'utilisation de signaux paracrines, les facteurs stimulateurs et inhibiteurs, comme agents pour promouvoir ou inhiber l'angiogenèse. Ces stratégies sont basées essentiellement sur l'utilisation de ces facteurs sous leur forme protéique comme agents stimulateurs ou inhibiteurs de l'angiogenèse, ou encore plus récemment sous la forme de vecteurs d'expression codant pour les facteurs choisis.

La découverte de nouvelles molécules pouvant servir de principe actif utile dans le traitement de pathologies liées à l'angiogenèse est rendue plus facile si l'on dispose de modèles d'études permettant de mener une expérimentation *in vitro.*

Il est possible de mettre en culture des cellules endothéliales. Une première méthode de culture consiste à mettre les cellules à adhérer sur le fond plat d'un flacon de culture, immergées dans un milieu de culture, puis à les incuber jusqu'à obtention d'un tapis cellulaire confluent. D'autres méthodes de culture se caractérisent par le fait de mettre les cellules endothéliales à adhérer sur le fond plat d'un flacon de culture préalablement couvert par une couche d'une protéine appartenant à la famille des protéines appelées protéines de la matrice extracellulaire. Dans d'autres méthodes encore, on recouvre les cellules endothéliales d'une lattice obtenue en rétractant une protéine de cette famille telle que le collagène ou la fibrine, formant ainsi respectivement une lattice de collagène et de fibrine.

Lors de la culture des cellules endothéliales, quelle que soit la technique mise en oeuvre, après un certain temps d'incubation on obtient une monocouche d'une population cellulaire homogène. Néanmoins, chaque population ainsi obtenue a une organisation sans rapport avec celle des cellules endothéliales différenciées formant des néovaisseaux.

Il a déjà été montré qu'il était possible lors de la culture des cellules endothéliales sous lattice de collagène ou de fibrine d'obtenir des cellules endothéliales différenciées formant des tubes capillaires *in vitro,* suite à la stimulation du tapis cellulaire de l'endothéliale par l'un des signaux paracrines promoteurs de l'angiogénèse (Montesano et al., Proc. Natl. Acad. Sci. USA, 1986, 83, 7297-7301). Ces tubes capillaires formés *in vitro* par les cellules endothéliales stimulées sont semblables aux néovaisseaux formées par le même type cellulaire in vivo lors de l'angiogénèse. Ils constituent donc un modèle adapté pour la recherche de nouveaux facteurs impliqués dans la régulation de l'angiogenèse.

La régulation de l'angiogenèse se fait à travers un équilibre entre l'action de facteurs stimulants et l'action de facteurs inhibants. Ces facteurs, qui sont eux-mêmes des produits de l'expression génétique, sont mitogéniques : ils contrôlent l'expression de plusieurs gènes, qui sont impliqués dans la régulation de l'angiogénèse et peuvent également être impliqués dans d'autres processus physiologiques ou pathologiques. Deux familles de gènes sont donc impliquées dans la régulation de l'angiogénès. D'une part, la famille de la première génération des gènes codant pour des facteurs stimulateurs ou inhibiteurs. D'autre part, la famille des gènes codant pour des constituants cellulaires intimement impliqués dans la régulation de l'angiogénèse, tels que des récepteurs cellulaires, des protéines de matrice extracellulaire, des protéines d'arrimage ("*docking proteins*"), des protéines de pontage ("*adaptator proteins*"), des kinases, des phosphatases, des protéases, des glycosyltransferases, etc... Cette famille de gènes constitue donc une "famille de la deuxième génération des gènes de l'angiogenèse".

La présente invention concerne un procédé d'identification des gènes codant pour des constituants cellulaires intimement impliqués dans la régulation de l'angiogenèse et appartenant à la famille de la deuxième génération des gènes de l'angiogenèse. On entend par constituants cellulaires, des protéines, groupes de protéines ou assemblage de protéines, du type défini ci-dessous pour les produits codés par les gènes des familles de la deuxième génération. Les facteurs stimulant ou inhibant le processus contrôlent l'expression de gènes impliqués dans l'augmentation ou l'inhibition de l'angiogenèse. Deux types de profils d'expression génétique doivent donc être étudiés : le premier est celui des cellules placées sous condition stimulatrice de l'angiogenèse, le second correspond à celui des cellules placées sous condition inhibitrice de l'angiogenèse.

Bien que l'état physiologique des cellules sous condition inhibitrice de l'angiogenèse et celui des cellules endothéliales non stimulées soit semblable, il est important de distinguer l'expression des gènes dans les deux cas. En effet, les cellules endothéliales stimulées avec un facteur favorisant l'angiogenèse, tel que le FGF1 par exemple, et incubées avec un facteur inhibiteur de l'angiogénèse ne sont pas capables de former des néovaisseaux. De plus, plusieurs facteurs inhibant l'angiogenèse sont des agents mitogéniques et affectent l'expression génétique tels que IL-10, IL-12, gro-α et β, etc.... Les cellules endothéliales peuvent donc présenter une expression génétique en condition inhibitrice de l'angiogenèse qui est différente de l'expression génétique d'une cellule endothéliale non stimulée. Il est donc important de comparer les profils de l'expression génétique des cellules lorsque celles-ci sont placées dans différentes conditions de culture, condition stimulatrice et condition inhibitrice de l'angiogenèse, par rapport à des conditions de culture de référence définies de façon précise.

L'identification de gènes codant pour des constituants cellulaires impliqués dans la régulation de l'angiogenèse est rendue possible grâce au procédé selon l'invention. Ce procédé est défini dans la revendication 1.

Pour identifier les membres de la famille de la deuxième génération des gènes impliqués dans la régulation de l'angiogénèse, les inventeurs ont donc déterminé quatre types de conditions expérimentales de culture des cellules endothéliales :
- les cellules cultivées sous condition de référence (CR) ne sont pas stimulées.
- les cellules cultivées sous condition promotrice d'angiogénèse (CPA) sont stimulées par un facteur angiogénique. Celui-ci peut être, par exemple, le FGF1, le FGF2, le HGF, le PDGF, etc....
- les cellules cultivées sous condition inhibitrice d'angiogenèse (CIA) sont stimulées par un facteur angiogénique et incubées avec un ou plusieurs facteurs anti-angiogéniques. Le facteur stimulant peut être le FGF2, le facteur inhibant peut, par exemple, être choisi parmi l'IL-10, l'IL-12, les chemokines gro-α ou β, etc...
- les cellules cultivées sous condition de contrôle (CC) sont incubées avec un facteur anti-angiogénique. Celui-ci peut être, par exemple, l'IL-10, l'IL-12, les chemokines gro-α ou β, etc...

Ces conditions de culture expérimentales sont appliquées à des cellules endothéliales cultivées selon l'un des procédés connus de l'homme du métier. La matrice de protéine extracellulaire peut notamment être constituée par de la fibrine, du collagène, de la laminine, du Matrigel, de la fibronectine ou tout autre protéine appartenant à la famille des protéines de la matrice extracellulaire.

Les facteurs capables de stimuler ou inhiber l'angiogenèse sont choisis parmi les facteurs dont l'action sur le processus de l'angiogenèse a été démontrée (Jensen, Surg. Neural., 1998, 49, 189-195 ; Tamatani et al., Carcinogenesis, 1999, 20, 957-962 ; Tanaka et al., Cancer Res., 1998, 58, 3362-3369 ; Ghe et al., Cancer Res., 1997, 57, 3733-3740 ; Kawahara et al., Hepatology, 1998, 28, 1512-1517 ; Chandhuni et al., Cancer Res., 1997, 57, 1814-1819 ; Jendraschak et Sage, Semin. Cancer Biol., 1996, 7, 139-146 ; Majewski et al., J. Invest. Dermatol., 1996, 106, 1114-1119).

Les facteurs stimulant l'angiogenèse mis en oeuvre à l'étape a) du procédé selon l'invention sont choisis parmi :
- les facteurs de croissance de fibroblaste 1 à 15 (FGF 1 à FGF 15)
- le facteur de croissance de l'épiderme (EGF)
- le facteur de croissance de l'endothéliale vasculaire (VEGF)
- le facteur de croissance de l'hépatocyte (HGF)
- le facteur de croissance dérivé de plaquette (PDGF)
- l'interleukine 8 (IL-8)
- l'angiogénine
- le facteur de croissance transformant (TGF).
- la neurokine midkine
- la pléiotropine
   de tout autre facteur de nature protéique inducteur de l'angiogenèse.

Les facteurs inhibant l'angiogenèse mis en oeuvre à l'étape a) du procédé selon l'invention sont choisis parmi :
- la thrombospondine
- l'angiostatine
- l'endostatine
- le facteur plaquettaire 4
- l'interleukine 10 (IL-10)
- l'interleukine 12 (IL-12)
- les chimioquines gro-α et β
- l'inhibiteur dérivé de chondrocyte humaine
- le facteur inhibiteur de la leucémie.
- le facteur tumoral de nécrose (TNF)
ou tout autre facteur de nature protéique inhibiteur de l'angiogenèse.

Les différents facteurs régulant l'angiogenèse sont additionnés aux cultures cellulaires selon des procédures bien connues de l'homme du métier (Montesano et al., Proc. Natl. Acad. Sci. USA, 1986, 83, 7297-7301). Les concentrations efficaces à employer pour chacun des différents facteurs sont avantageusement les suivantes : de 1 ng/ml à 200 ng/ml.

Dans le procédé selon l'invention, il est possible que un ou plusieurs des facteurs capables de stimuler ou d'inhiber l'angiogenèse soi(en)t ajouté(s) aux cellules en culture sous la forme d'un vecteur d'expression construit de manière à permettre la synthèse du ou des facteurs dans la culture cellulaire. Un exemple d'un tel vecteur est cité dans Tanaka et al. (Cancer Res., 1998, 58, 3362-3369).

Le procédé selon l'invention comprend, à l'étape c) l'analyse du profil d'expression des gènes des cellules sous condition stimulatrice ou inhibitrice d'angiogenèse. Cette analyse est fondée sur l'étude des ARN messagers des cellules endothéliales sous condition stimulatrice ou inhibitrice d'angiogenèse, par comparaison avec les ARN messager des cellules sous condition de référence et de contrôle. Ces ARN messager sont extraits des cellules par des techniques classiques (Vancopoulos et al., 1990, dans "Methods for cloning and analysis of eucaryotic genes", p 8-23, Ed. Jones and Bartlett, Boston) qui permettent d'isoler les ARN des autres constituants cellulaires. Les ARN messagers sont isolés des autres ARN, par exemple en tirant parti de la présence de la séquence poly-A présente à leur extrémité.

L'analyse des différentes populations d'ARN messager peut être réalisée par une méthode d'affichage différentiel comprenant les étapes suivantes :
- les ARN messagers sont rétro-transcrits
- les molécules d'ADN obtenu par rétro-transcription sont séparées par électrophorèse
- les molécules d'ADN d'intérêt sont détectées puis extraites du support l'électrophorèse,
- l'ADN d'intérêt est purifié
- l'ADN purifié peut être utilisé comme sonde pour cribler une banque d'ADNc, il peut aussi être directement sous cloné et séquencé.

L'ADN purifié peut en particulier être utilisé comme une sonde pour cribler une banque d'ADNc, ou bien pour détecter un acide nucléique complémentaire par Northern blot.

L'affichage différentiel permet la détection simultanée de deux groupes de gènes exprimés différentiellement, par exemple certains gènes liés à un facteur angiogénique choisi parmi (a), et d'autres liés à un facteur anti-angiogénique choisi parmi (b). La technique d'affichage différentiel permet aussi de détecter des ARNm rares et de minimiser la redondance et les clones de faux positifs.

Dans le procédé selon l'invention, l'analyse des différentes populations d'ARN messager peut également être réalisée par hybridation soustractive.

L'hybridation soustractive inclut l'isolement des ARNm, la rétro-transcription des ARNm, la construction de bibliothèques d'ADNc, la soustraction, et le criblage par hybridation différentielle.

Les deux techniques sont complémentaires et seront utilisées pour identifier les ARNm en rapport avec l'angiogénèse.

Le procédé selon l'invention est donc remarquable en ce qu'il permet l'appréciation qualitative et quantitative de l'angiogénèse, sous l'effet de deux types de signaux paracrines, angiogéniques et anti-angiogéniques.

En outre, ce procédé permet de tester l'effet de différentes protéines comme matrice extracellulaire (fibrine, collagène, laminine, Matrigel, fibronectine, etc...) en combinaison avec les signaux paracrines. De plus, le procédé peut être facilement modifié pour inclure un autre type cellulaire en plus des cellules endothéliales.

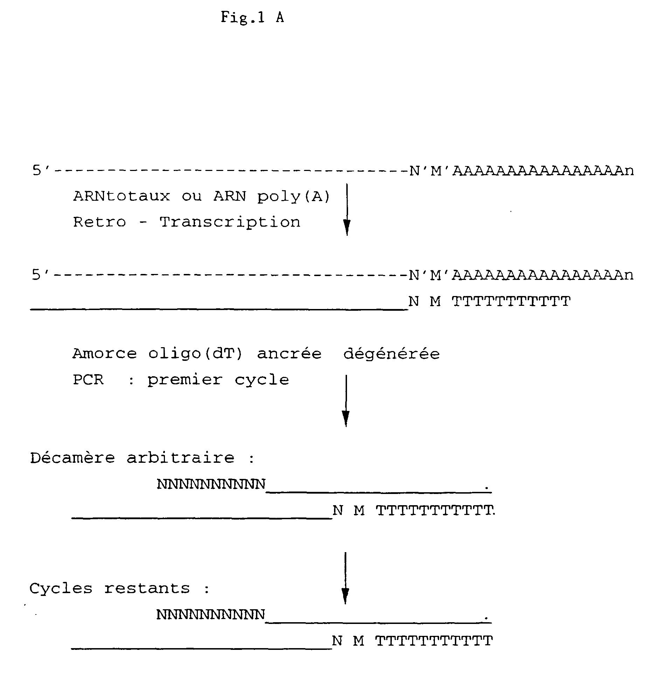

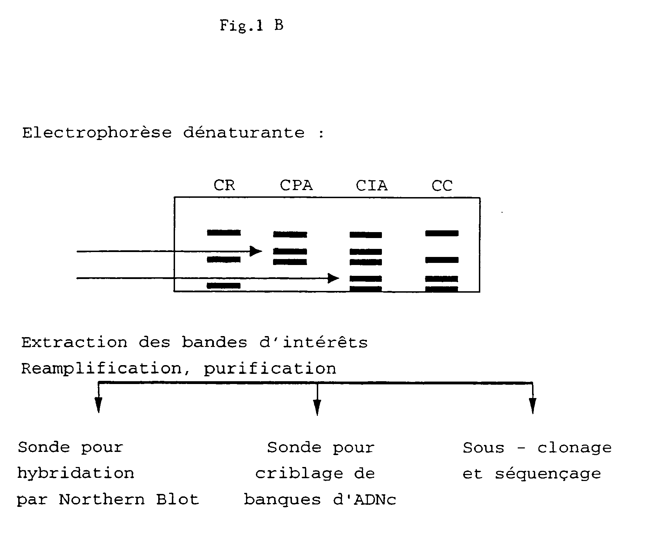

D'autres avantages et caractéristiques de l'invention apparaîtront à la lecture des exemples qui suivent, donnés à titre non limitatif et se référant à la figure 1.

### Exemple 1 : affichage différentiel:

La figure 1 représente schématiquement la méthode de l'affichage différentiel de gènes impliqués dans l'angiogénèse.

Les ARNm sont extraits spécifiquement et rétro-transcrits en utilisant chacun des quatre groupes dégénérés d'amorces oligo(dT), T12MN, dans lequel M peut être G, A, ou C; et N peut être G, A, T, ou C.

Chaque groupe d'amorces est défini par la base de l'extrémité 3' (N), avec une dégénérescence au niveau de la position (M). Par exemple, le jeu d'amorces dans lequel N=G est constitué par:
5'-TTTTTTTTTTTTGG-3'
5'-TTTTTTTTTTTTAG-3'
5'-TTTTTTTTTTTTCG-3'

Le diagramme du gel schématise les résultats de l'utilisation d'un seul groupe de *primaire* avec les cellules endothéliales sous les trois conditions expérimentales : CR = Condition de Référence (cellules endothéliales en culture sans aucune stimulation), CPA = Condition Promotrice d'Angiogenèse (cellules endothéliales stimulées par un facteur angiogénique choisi parmi (a), tel que le FGF2), CIA = Condition Inhibitrice de l'Angiogenèse (cellules endothéliales sont stimulées par un facteur angiogénique parmi (a) et un facteur anti-angiogénique parmi (b) simultanément) ; CC = Condition de Contrôle (cellules endothéliales stimulées par un facteur anti-angiogénique choisi parmi (b)) .

Les lignes en pointillés de la figure 1 représentent l'ARN, les lignes continues représentent l'ADN. T12MN est l'amorce dégénérée d'oligo(dT); M peut être A, G, ou G (position dégénérée); N peut être A, C, G, ou T.

### Exemple 2 : hybridation soustractive

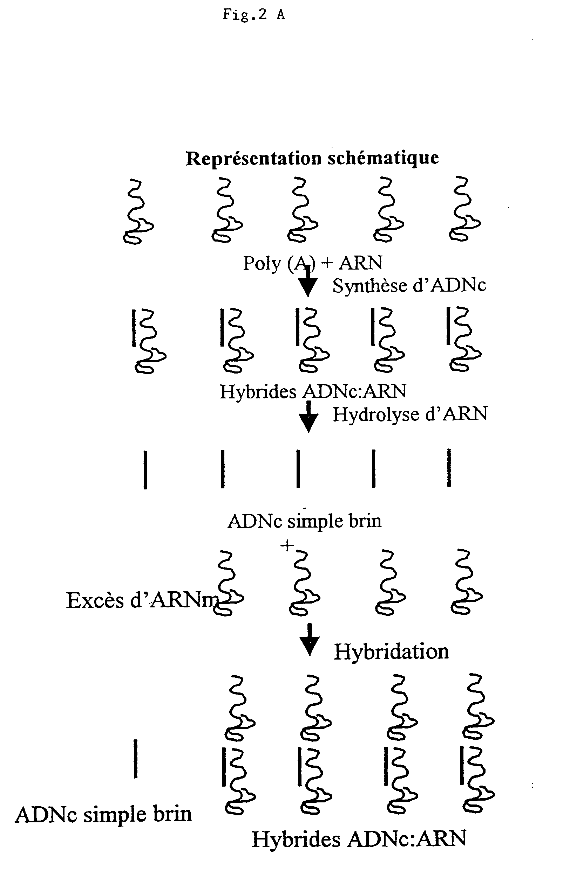

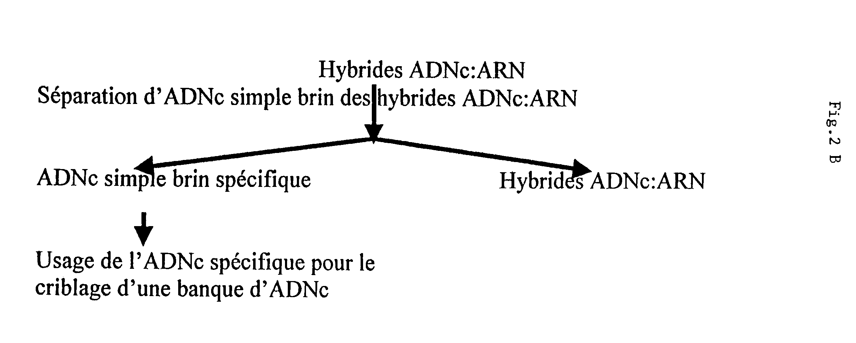

La figure 2 représente schématiquement la méthode de l'hybridation soustractive d'ADNc.

Les ARNm sont extraits de cellules placées sous différentes conditions opératoires et rétro-transcrits, afin d'obtenir les ADNc simple brin représentant les séquences des ARN exprimés dans lesdites cellules.

L'ADNc simple brin issu d'une cellule placée sous une condition opératoire, par exemple une condition stimulatrice d'angiogenèse, est hybridé avec un excès d'ARN issu de cellules placées sous une autre condition opératoire, par exemple les conditions de référence. Dans telle réaction d'hybridation, l'ADNc correspondant à des séquences exprimées dans les deux conditions opératoires formera des hybrides avec l'ARN. Au contraire, l'ADNc qui n'est pas représenté dans l'ARN restera sous forme d'ADNc simple brin.

Avantageusement, en limitant la quantité d'ARNm utilisée dans la réaction d'hybridation, cette méthode peut être aussi utilisée pour isoler une séquence ADNc représentant un ou plusieurs ARNm sur-exprimés dans les cellules placées sous une condition opératoire donnée, par exemple une condition stimulatrice d'angiogenèse.

L'ADNc double brin et l'ADNc simple brin peuvent être séparés, notamment par chromatographie sur une colonne d'hydroxyapatite. L'ADNc simple brin ainsi isolé correspond à un ARNm spécifiquement présent ou surexprimé dans des cellules placées sous une condition particulière de culture. Cet ADNc peut être utilisé pour cribler une banque d'ADNc afin d'identifier, isoler et cloner le gène correspondant.

## Revendications

1. Procédé d'identification de gènes codant pour des constituants cellulaires impliqués dans la régulation de l'angiogenèse, **caractérisé en ce qu'**il comprend les étapes suivantes :
a) la culture de cellules endothéliales sur une protéine de la matrice extracellulaire, selon quatre types de conditions différentes :
- une condition de référence dans laquelle les cellules ne sont pas stimulées ;
- une condition promotrice d'angiogénèse dans laquelle les cellules sont stimulées par un facteur angiogénique ;
- une condition inhibitrice d'angiogenèse dans laquelle les cellules sont stimulées par un facteur angiogénique et incubées avec un ou plusieurs facteurs anti-angiogéniques ;
- une condition de contrôle dans laquelle les cellules sont incubées avec un facteur anti-angiogénique
b) l'isolement des ARN messager provenant des cellules cultivées selon les différentes conditions
c) la comparaison sur le plan qualitatif et/ou quantitatif des différentes populations d'ARN messager, afin d'identifier les ARN messagers provenant exclusivement, ou en quantité particulièrement élevée, de cultures cellulaires sous condition stimulatrice et/ou sous condition inhibitrice de l'angiogenèse, correspondant aux gènes codant pour des constituants cellulaires impliqués dans la régulation de l'angiogenèse.

2. Procédé selon la revendication 1, **caractérisé en ce qu**'il comprend en outre les étapes suivantes :
- l'isolement des ARN messagers identifiés à l'étape c), leur amplification et leur purification
- le clonage et le séquençage des molécules d'acide nucléique obtenues lors de l'étape précédente
- l'identification du ou des gènes correspondant aux molécules d'acide nucléique isolées.

3. Procédé selon l'une des revendications 1 ou 2, **caractérisé en ce que** la matrice extracellulaire est constituée par de la fibrine, du collagène, de la laminine, du Matrigel®, de la fibronectine ou tout autre protéine appartenant à la famille des protéines de la matrice extracellulaire.

4. Procédé selon l'une des revendications 1 à 3, **caractérisé en ce que** les facteurs stimulant l'angiogenèse mis en oeuvre à l'étape (a) sont choisis parmi :
- les facteurs de croissance de fibroblaste 1 à 15 (FGF1 à FGF15);
- le facteur de croissance de l'épiderme (EGF)
- le facteur de croissance de l'endothélium vasculaire (VEGF);
- le facteur de croissance de l'hépatocyte (HGF)
- le facteur de croissance dérivé de plaquette (PDGF)
- l'interleukine 8 (IL-8)
- l'angiogénine
- le facteur de croissance transformant (TGF)
- la neurokine midkine
- la pléiotropine
ou tout autre facteur de nature protéique, inducteur de l'angiogenèse.

5. Procédé selon l'une des revendications 1 à 4, **caractérisé en ce que** les facteurs inhibant l'angiogenèse mis en oeuvre à l'étape a) du procédé selon l'invention sont choisis parmi
- la thrombospondine
- l'angiostatine
- l'endostatine
- le facteur plaquettaire 4
- l'interleukine 10 (IL-10)
- l'interleukine 12 (IL-12)
- les chimioquines gro-α et β
- l'inhibiteur dérivé de chondrocyte humain
- le facteur inhibiteur de la leucémie
- le facteur tumoral de nécrose (TNF)
ou tout autre facteur de nature protéique, inhibiteur de l'angiogenèse.

6. Procédé selon l'une des revendications 1 à 5, **caractérisé en ce que** un ou plusieurs des facteurs capables de stimuler ou d'inhiber l'angiogenèse sont ajoutés aux cellules en culture sous la forme d'un vecteur d'expression construit de manière à permettre la synthèse du ou des facteurs dans la culture cellulaire.

7. Procédé selon l'une des revendications 1 à 6, **caractérisé en ce que** l'analyse des différentes populations d'ARN messager est réalisée par une méthode d'affichage différentiel comprenant les étapes suivantes :
- les ARN messagers sont rétro-transcrits
- les molécules d'ADN obtenu par rétro-transcription sont séparées par électrophorèse
- les molécules d'ADN d'intérêt sont détectées puis extraites du support l'électrophorèse,
- l'ADN d'intérêt est purifié
- l'ADN purifié est éventuellement utilisé comme sonde,
- l'ADN purifié est éventuellement sous cloné et séquencé.

8. Procédé selon l'une des revendications 1 à 7, **caractérisé en ce que** l'analyse des différentes populations d'ARN messager est réalisée par hybridation soustractive.

## Claims

1. Method for identifying genes coding for cellular constituents involved in the regulation of angiogenesis, **characterised in that** it includes the following steps:
a) the culture of endothelial cells on a protein of the extracellular matrix, according to four different conditions:
- a reference condition in which the cells are not stimulated;
- an angiogenesis-promoting condition in which the cells are stimulated by an angiogenic factor;
- an angiogenesis-inhibiting condition in which the cells are stimulated by an angiogenic factor and incubated with one or more anti-angiogenic factors;
- a control condition in which the cells are incubated with an anti-angiogenic factor,
b) the isolation of messenger RNAs coming from cells cultivated according to the different conditions,
c) the qualitative and/or quantitative comparison of the different messenger RNA populations so as to identify the messenger RNAs coming exclusively, or in a particularly high amount, from cell cultures under a angiogenesis-stimulating condition and/or an angiogenesis-inhibiting condition, corresponding to the genes coding for cellular constituents involved in the regulation of angiogenesis.

2. Method according to claim 1, **characterised in that** it also includes the following steps:
- isolation of the messenger RNAs identified in step (c), and the amplification and purification thereof,
- cloning and sequencing of the nucleic acid molecules obtained in the prepeding step,
- identification of the gene(s) corresponding to the isolated nucleic acid molecules.

3. Method according to one of claims 1 or 2, **characterised in that** the extracellular matrix consists of fibrin, collagen, laminin, Matrigel®, fibronectin or any other protein belonging to the family of proteins of the extracellular matrix.

4. Method according to one of claims 1 to 3, **characterised in that** the factors stimulating angiogenesis implemented in step (a) are selected from:
- fibroblast growth factors 1 to 15 (FGF1 to FGF15);
- epidermal growth factor (EGF);
- vascular endothelial growth factor (VEGF);
- hepatocyte growth factor (HGF);
- platelet-derived growth factor (PDGF);
- interleukin 8 (IL-8);
- angiogenin;
- transforming growth factor (TGF);
- neurokine midkine;
- pleiotropin;
or any other protein factor promoting angiogenesis.

5. Method according to one of claims 1 to 4, **characterised in that** the angiogenesis-inhibiting factors implemented in step (a) of the method according to the invention are selected from:
- thrombospondin
- angiostatin
- endostatin
- platelet factor 4
- interleukin 10 (IL-10)
- interleukin 12 (IL-12)
- chemokines gro-α and β
- human chondrocyte-derived inhibitor
- leukaemia inhibitory factor
- tumour necrosis factor (TNF)
or any other angiogenesis-inhibiting protein factor.

6. Method according to one of claims 1 to 5, **characterised in that** one or more of the factors capable of stimulating or inhibiting angiogenesis are added to the cells in culture in the form of an expression vector constructed so as to enable the synthesis of the factor(s) in the cell culture.

7. Method according to one of claims 1 to 6, **characterised in that** the analysis of the different messenger RNA populations is performed using a differential display method including the following steps:
- the messenger RNAs are retro transcribed;
- the DNA molecules obtained by retrotranscription are separated by electrophoresis;
- the DNA molecules of interest are detected, then extracted from the electrophoresis support;
- the DNA of interest is purified;
- the purified DNA is optionally used as a probe;
- the purified DNA is optionally subcloned and sequenced.

8. Method according to one of claims 1 to 7, **characterised in that** the analysis of the different messenger RNA populations is performed by subtractive hybridisation.

## Patentansprüche

1. Verfahren zur Identifizierung von codierenden Genen für zellulare Bestandteile, die in der Regelung der Angiogenese impliziert sind, **dadurch gekennzeichnet, dass** es die folgenden Schritte umfasst:
a) die Kultur von endothelialen Zellen auf einem Protein der exträzellularen Matrix, nach vier verschiedenen Bedingungstypen:
- eine Bezugsbedingung in der die Zellen nicht stimuliert sind;
- eine die Angiogenese fördernde Bedingung, in der die Zellen durch einen angiogenischen Faktor stimuliert sind;
- eine die Angiogenese inhibierende Bedingung, in der die Zellen durch einen angiogenischen Faktor stimuliert sind und mit einem oder mehreren anti-angiogenischen Faktor(en) inkubiert sind;
- eine Kontrollbedingung in der die Zellen mit einem anti-angiogenischen Faktor inkubiert sind;
b) das Isolieren der Boten-RNA, die von den Zellen stammen, die nach den verschiedenen Bedingungen kultiviert sind;
c) das Vergleichen in Bezug auf die Qualität und/oder die Quantität der verschiedenen Populationen von Boten-RNA, um die Boten-RNA zu identifizieren, die exklusiv oder in besonders hoher Menge von zellularen Kulturen unter stimulierender Bedingung und/oder unter inhibierender Bedingung der Angiogenese stammen, die den codierenden Genen für zellulare Bestandteile entspricht, die in der Regelung der Angiogenese impliziert sind.

2. Verfahren nach Anspruch 1, **dadurch gekennzeichnet, dass** es darüber hinaus die folgenden Schritte umfasst:
- das Isolieren der Boten-RNA, die im Schritt c) identifiziert werden, deren Amplifikation und deren Reinigung;
- das Klonen und die Blockbildung der NukleinsäureMoleküle, die anlässlich des vorhergehenden Schritts erhalten werden;
- die Identifizierung des oder der Gene, die den isolierten Nukleinsäure-Molekülen entsprechen.

3. Verfahren nach einem der Ansprüche 1 oder 2, **dadurch gekennzeichnet, dass** sich die extrazellulare Matrix wie folgt zusammensetzt: durch Fibrin, Kollagen, Laminin, Matrigel®, Fibronectin oder jedes andere Protein, das zu der Familie der Proteine von der extrazellularen Matrix gehört.

4. Verfahren nach einem der Ansprüche 1 bis 3, **dadurch gekennzeichnet, dass** die die Angiogenese stimulierenden Faktoren, die im Schritt (a) eingesetzt werden, zwischen den Folgenden ausgewählt werden:
- die Wachstumsfaktoren von Fibroblast 1 bis 15 (FGF1 bis FGF15);
- der epidermale Wachstumsfaktor (EGF)
- der vaskulo endotheliale Wachstumsfaktor (VEGF);
- der Hepatozyten Wachstumsfaktor (HGF)
- der Plättchen abhängige Wachstumsfaktor (PDGF)
- das Interleukin 8 (IL-8)
- das Angiogenin
- der transformierende Wachstumsfaktor (TGF)
- das Neurokin Midkin
- die Pleiotropin
oder jeder andere proteinartige Faktor, der die Angiogenese induziert.

5. Verfahren nach einem der Ansprüche 1 bis 4, **dadurch gekennzeichnet, dass** die die Angiogenese inhibierenden Faktoren, die im Schritt a) des Verfahrens nach der Erfindung eingesetzt werden, zwischen den Folgenden ausgewählt werden:
- das Thrombospondin
- das Angiostatin
- das Endostatin
- der Plättchen Faktor 4
- das Interleukin 10 (IL-10)
- das Interleukin 12 (IL-12)
- die Chemokine GRO-alpha und beta
- der von menschlichem Chondrocyt abhängige Inhibitor
- der inhibierende Faktor der Leukämie
- der Tumor-Nekrose-Faktor (TNF)
oder jeder andere proteinartige Faktor, der die Angiogenese inhibiert.

6. Verfahren nach einem der Ansprüche 1 bis 5, **dadurch gekennzeichnet, dass** einer oder mehrere von den Faktoren, die fähig sind, die Angiogenese zu stimulieren oder zu inhibieren, den Zellen in Kultur hinzugefügt sind, und dies in der Form eines Ausdrucksvektors, der derart gebildet ist, dass er die Synthese von dem Faktor oder von den Faktoren in der zellularen Kultur erlaubt.

7. Verfahren nach einem der Ansprüche 1 bis 6, **dadurch gekennzeichnet, dass** die Analyse der verschiedenen Populationen von Boten-RNA durch ein Verfahren der Differenzialanzeige ausgeführt wird, das die folgenden Schritte umfasst:
- die Boten-RNA sind retrotranskribiert,
- die durch Retrotranskription erhaltenen ADN Moleküle werden durch Elektrophorese getrennt,
- die interessanten ADN Moleküle werden erkannt und anschließend vom Träger der Elektrophorese extrahiert,
- das interessante ADN wird gereinigt,
- das gereinigte ADN wird ggf. als Sonde verwendet,
- das gereinigte ADN wird ggf. untergeklont und sequenziert.

8. Verfahren nach einem der Ansprüche 1 bis 7, **dadurch gekennzeichnet, dass** die Analyse der verschiedenen Populationen an Boten-RNA durch die substraktive Hybridisierung realisiert wird.
